# FASCICULE DE BREVET EUROPEEN

(11) **EP 1 223 933 B1**
(45) Date de publication et mention de la délivrance du brevet: **06.09.2006**
(21) Numéro de dépôt: 00967988.7
(22) Date de dépôt: 10.10.2000
(51) Int. Cl.: A61K 31/426, A61K 31/421, A61K 31/42, A61K 31/417, A61P 25/00, C07D 277/28

(54) **DÉRIVÉS D'HÉTÉROCYCLES À 5 CHAINONS ET LEUR APPLICATION COMME INHIBITEURS DE MONOAMINE OXYDASE**
FÜNFGLIEDRIGE DERIVATE VON HETEROZYKLEN UND IHRE VERWENDUNG ALS MONOAMINOXIDASE-INHIBITOREN
5-MEMBERED HETEROCYCLE DERIVATIVES AND USE THEREOF AS MONOAMINOXIDASE INHIBITORS

(30) Priorité: 11.10.1999 FR 9912643; 01.08.2000 FR 0010151; 01.09.2000 FR 0011169
(43) Date de publication de la demande: 24.07.2002
(60) Demande divisionnaire: 02076763.8
(73) Titulaire: SOCIETE DE CONSEILS DE RECHERCHES ET D'APPLICATIONS SCIENTIFIQUES (S.C.R.A.S.), 75016 Paris (FR)
(72) Inventeur: CHABRIER de LASSAUNIERE, Pierre-Etienne, F-75016 Paris (FR); HARNETT, Jeremiah, F-91190 Gif sur Yvette (FR); BIGG, Dennis, F-91190 Gif-sur-Yvette (FR); POMMIER, Jacques, F-92700 Colombes (FR); LANNOY, Jacques, Résidence Le Renouveau, Bâtim. B, F-91470 Bièvres (FR); LIBERATORE, Anne-Marie, F-78610 Auffargis (FR); THURIEAU, Christophe, F-75016 Paris (FR)
(74) Mandataire: Bourgouin, André
(86) Numéro de dépôt international: PCT/FR2000/002805
(87) Numéro de publication internationale: WO 2001/026656

(56) Documents cités:
- EP-A- 0 432 740
- EP-A- 0 908 180
- WO-A-00/39130
- WO-A-96/16040
- WO-A-98/15274
- WO-A-98/27108
- WO-A-98/58934
- WO-A-99/64401
- WO-A-99/64420
- UNANGST P C ET AL: "Novel 1,2,4-oxadiazoles and 1,2,4-thiadiazoles as dual 5-lipoxygenase and cyclooxygenase inhibitors" JOURNAL OF MEDICINAL CHEMISTRY, vol. 35, no. 20, 2 octobre 1992 (1992-10-02), pages 3691-8, XP002142240

## Description

La présente invention concerne l'utilisation du 4-[2-(aminométhyl)-1,3-thiazol-4-yl]-2,6-di(*tert*-butyl)phénol pour préparer un médicament destiné à inhiber les monoamine oxydases (MAO) et/ou peroxydation lipidique et/ou à agir en tant que modulateurs des canaux sodiques. Elle a également pour objet, en tant que médicaments, le composé 4-[2-(aminométhyl)-1,3-thiazol-4-yl]-2,6-di(*tert*-butyl)phénol. Elle concerne de plus le 4-[2-(aminométhyl)-1,3-thiazol-4-yl]-2,6-di(*tert*-butyl)phénol.

En effet, compte tenu du rôle potentiel des MAO et des ROS («*reactive oxygen species* » ou espèces réactives de l'oxygène, à l'origine de la peroxydation lipidique) en physiopathologie, le 4-[2-(aminométhyl)-1,3-thiazol-4-yl]-2,6-di(*tert*-butyl)phénol peut produire des effets bénéfiques ou favorables dans le traitement de pathologies où ces enzymes et/ou ces espèces radicalaires sont impliquées. Notamment :
- les troubles du système nerveux central ou périphérique comme par exemple les maladies neurologiques où l'on peut notamment citer la maladie de Parkinson, les traumatismes cérébraux ou de la moelle épinière, l'infarctus cérébral, l'hémorragie sub arachnoïde, l'épilepsie, le vieillissement, les démences séniles, la maladie d'Alzheimer, la chorée de Huntington, la sclérose latérale amyotrophique, les neuropathies périphériques, la douleur ;
- la schizophrénie, les dépressions, les psychoses ;
- les troubles de la mémoire et de l'humeur ;
- les pathologies comme par exemple la migraine ;
- les troubles du comportement, la boulimie et l'anorexie ;
- les maladies auto-immunes et virales comme par exemple le lupus, le sida, les infections parasitaires et virales, le diabète et ses complications, la sclérose en plaques.
- l'addiction aux substances toxiques ;
- les pathologies inflammatoires et prolifératives ;
- et plus généralement toutes les pathologies caractérisées par une production excessive des ROS et/ou une participation des MAO.

Dans l'ensemble de ces pathologies, il existe des évidences expérimentales démontrant l'implication des ROS *(Free Radic. Biol. Med.* (1996) **20**, 675-705 ; *Antioxid. Health. Dis.* (1997) **4** (Handbook of Synthetic Antioxidants), 1-52) ainsi que l'implication des MAO (Goodman & Gilman's : *The pharmacological basis of therapeutics ,* 9th ed., 1995, 431-519).

L'intérêt d'une combinaison des activités inhibitrice de MAO et inhibitrice de la peroxydation lipidique est par exemple bien illustré dans la maladie de Parkinson. Cette pathologie est caractérisée par une perte des neurones dopaminergiques de la voie nigrostriatal dont la cause serait en partie liée à un stress oxydatif dû aux ROS. De la dopamine exogène à partir de L Dopa est utilisé en thérapeutique afin de maintenir des taux suffisants de dopamine. Les inhibiteurs de MAO sont aussi utilisés avec la L Dopa pour éviter sa dégradation métabolique mais n'agissent pas sur les ROS. Des composés agissant à la fois sur les MAO et les ROS auront donc un avantage certain.

Par ailleurs, le caractère de modulateur des canaux sodiques est très utile pour des indications thérapeutiques comme :
- le traitement ou la prévention de la douleur, et notamment :
   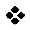 des douleurs post-opératoires,
   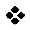 de la migraine,
   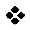 des douleurs neuropathiques telles que la névralgie du trijumeau, la douleur post-herpétique, les neuropathies diabétiques, les névralgies glossopharyngiennes, les radiculopathies et neuropathies secondaires à des infiltrations métastatiques, l'adiposis dolorosa et les douleurs liées aux brûlures,
   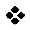 des douleurs centrales consécutives aux accidents cérébraux vasculaires, lésions thalamiques et sclérose en plaques, et
   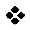 des douleurs chroniques inflammatoires ou liées à un cancer ;
- le traitement de l'épilepsie ;
- le traitement de troubles liés à la neurodégénération, et en particulier :
   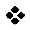 des accidents cérébraux vasculaires,
   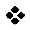 du traumatisme cérébral, et
   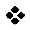 de maladies neurodégénératives telles que la maladie d'Alzheimer, la maladie de Parkinson et la sclérose latérale amyotrophique ;
- le traitement des troubles bipolaires et du syndrome du colon irritable.

Les avantages concrets de la présence dans le composé selon l'invention d'au moins une de ces activités ressortent donc clairement de ce qui précède.

La demande de brevet européen EP 432 740 décrit des dérivés d'hydroxyphénylthiazoles, lesquels peuvent être utilisés dans le traitement des maladies inflammatoires, en particulier les maladies rhumatismales. Ces dérivés d'hydroxyphénylthiazoles montrent des propriétés de piégeurs de radicaux libres et d'inhibiteurs du métabolisme de l'acide arachidonique (ils inhibent la lipoxygénase et la cyclooxygénase).

D'autres dérivés d'hydroxyphénylthiazoles ou d'hydroxyphényloxazoles sont décrits dans la demande de brevet PCT WO 99/09829. Ceux-ci possèdent des propriétés analgésiques.

Un certain nombre de dérivés d'imidazoles de structures proches à celle du composé selon l'invention ont par ailleurs été décrits par la demanderesse dans la demande de brevet PCT WO 99/64401 comme agonistes ou antagonistes de la somatostatine. Lesdits dérivés d'imidazoles possèdent cependant des propriétés thérapeutiques dans des domaines différents de ceux indiqués ci-dessus (la suppression de l'hormone de croissance et le traitement de l'acromégalie, le traitement de la resténose, l'inhibition de la sécrétion d'acide gastrique et la prévention des saignements gastro-intestinaux notamment).

Par ailleurs, les composés de formule générale **(A1)** 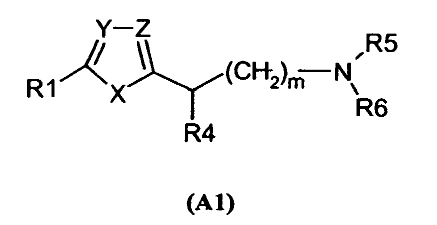 dans laquelle
R1 représente l'un des radicaux aryle, hétéroaryle, aralkyle ou cycloalkyle optionnellement substitués par un à trois substituants choisis indépendamment parmi un atome halogène, le radical CF₃, CN, OH, alkyle ou alkoxy, SO₂R9 avec R9 représentant NH₂ ou NHCH₃;
X représente NR2, R2 représentant H ou alkyle ;
Y représente N ou CR3 ;
Z représente CR3 ou N ;
à la condition toutefois que Y et Z ne sont pas tous deux CR3 ou N en même temps ;
R3 représente H, alkyle, halogène, hydroxyalkyle ou phényle éventuellement substitué par 1 à 3 subtituants choisis parmi H, CF₃, CN, SO₂NH₂, OH, alkyle ou alkoxy ;
m représente 0, 1 ou 2 ;
R4 représente H ou alkyle ;
lorsque Z représente CR3, alors R3 et R4 peuvent aussi représenter ensemble -(CH₂)ₙ₁- avec n1 entier de 2 à 4 ou R2 et R4 peuvent aussi représenter ensemble -(CH₂)ₙ₂- avec n2 entier de 2 à 4 ;
R5 et R6 représentent indépendamment H, alkyle, alkoxy, aryle ou aralkyle ;
NR5R6 pouvant aussi représenter ensemble (notamment) :
   - le radical 2-(1,2,3,4-tétrahydroquinolyl) éventuellement substitué,
   - un radical 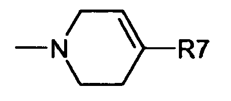 dans lequel R7 représente l'un des radicaux phényle, benzyle ou phénéthyle dans lequels le cycle phényle peut être substitué ;
   - un radical 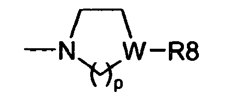 dans lequel p est un entier de 1 à 3,
W est N et R8 représente H, CF₃, l'un des radicaux phényle, pyridyle ou pyrimidinyle éventuellement substitués de 1 à 2 fois par des radicaux choisis parmi halogène, OH, alkyle ou alkoxy, ou
W est CH et R8 représente phényle éventuellement substitué ou aralkyle éventuellement substitué sur le groupe aryle ;
ont été décrits dans la demande de brevet PCT WO 96/16040 en tant qu'agonistes partiels ou antagonistes des sous-récepteurs de la dopamine du cerveau ou en tant que formes prodrogues de tels agonistes partiels ou antagonistes. Ces composés présenteraient de ce fait des propriétés intéressantes dans le diagnostic et le traitement de désordres affectifs tels que la schizophrénie et la dépression ainsi que certains désordres du mouvement tels que la maladie de Parkinson.

Il a également été décrit dans la demande de brevet PCT WO 98/27108 que certains amides de formule générale **(A2)** 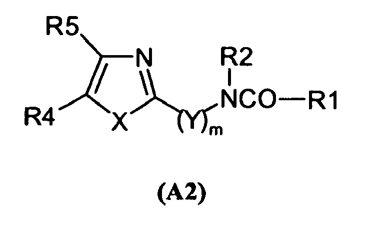 dans laquelle :
R1 représente notamment un radical alkyle, phényle éventuellement substitué ou aryle hétérocyclique éventuellement substitué ;
R2 représente H ou phénylalkyle ;
R4 représente H, quinolyle, 3-4-méthylènedioxyphényle ou l'un des radicaux phényle ou pyridyle éventuellement substitués, par un ou des radicaux choisis notamment parmi alkyle, alkoxy, alkylthio, hydroxy éventuellement protégé, amino, alkylamino, dialkylamino ;
R5 représente H ou un radical imidazolyle, phényle, nitrophényle, phénylalkyle, ou encore un radical -CO-N(R7)(R8), dans lequel R7 et R8 représentent indépendamment H, phényle, phénylalkyle, alkyle ou alkoxy ;
ou R4 et R5 en combinaison forment un groupe de formule -CH=CH-CH=CH- ;
Y est un radical phénylène substitué par un radical phényle, phénoxy ou phénylalkoxy, ou un groupe de formule -CH(R3)-, dans laquelle R3 représente H ou un radical de formule -(CH₂)ₙ-R6, dans laquelle R6 représente un radical hydroxy éventuellement protégé, acyle, carboxy, acylamino, alkoxy, phénylalkoxy, alkylthio, phényle éventuellement substitué, pyridyle éventuellement substitué, pyrazinyle, pyrimidinyle, furyle, imidazolyle, naphtyle, N-alkylindolyle ou 3,4-méthylènedioxyphényle et n est un entier de 0 à 3 ;
R2 et R3 pris ensemble avec les atomes de carbone qui les portent pouvant former un groupe phényle ;
X représente S ou NR9 ;
R9 représentant H, un radical alkyle ou cycloalkyle, ou encore un radical benzyle éventuellement substitué une fois sur sa partie phényle par H, alkyle ou alkoxy ;
sont des inhibiteurs des NO synthases et peuvent être utilisés pour traiter des maladies qui comprennent notamment l'ischémie cardiovasculaire ou cérébrale, l'hémorragie cérébrale, les troubles du système nerveux central, la maladie d'Alzheimer, la sclérose en plaques, le diabète, l'hépatite, la migraine, l'arthrite rhumatoïde et l'ostéoporose.

Dans un domaine différent, la demanderesse a elle-même précédemment décrit dans la demande de brevet PCT WO 98/58934 des dérivés d'amidines ayant la faculté d'inhiber les NO Synthases et/ou la peroxydation lipidique.

La demanderesse a maintenant découvert de façon surprenante que le 4-[2-(aminométhyl)-1,3-thiazol-4-yl]-2,6-di(*tert*-butyl)phénol, possède au moins l'une des trois propriétés choisies parmi les propriétés suivantes (et souvent même deux de ces trois propriétés voire parfois les trois à la fois) :
- des propriétés d'inhibition des MAO ;
- des propriétés d'inhibition de la peroxydation lipidique ; et
- des propriétés de modulation des canaux sodiques.

Ces propriétés avantageuses offrent l'intérêt d'ouvrir à ce composé selon l'invention de nombreuses applications, en particulier dans le traitement des maladies neurodégénératives, et notamment celles indiquées précédemment, de la douleur ou de l'épilepsie.

Selon l'invention, le composé est le 4-[2-(aminométhyl)-1,3-thiazol-4-yl]-2,6-di(*tert-*butyl)phénol ou son sel pharmaceutiquement acceptable.

Le composé selon l'invention possède au moins deux des activités citées ci-dessus. En particulier, il a à la fois une activité antagoniste vis-à-vis des canaux sodiques et une activité piégeuse des ROS.

Ceci permet au composé selon l'invention d'être utile dans le traitement des maladies citées précédemment comme étant reliées aux MAO, à la peroxydation lipidique et aux canaux sodiques.

Par alkyle, lorsqu'il n'est pas donné plus de précision, on entend un radical alkyle linéaire ou ramifié comptant de 1 à 6 atomes de carbone. Par cycloalkyle, lorsqu'il n'est pas donné plus de précision, on entend un système monocyclique carboné comptant de 3 à 7 atomes de carbone. Par alkényle, lorsqu'il n'est pas donné plus de précision, on entend un radical alkyle linéaire ou ramifié comptant de 1 à 6 atomes de carbone et présentant au moins une insaturation (double liaison). Par alkynyle, lorsqu'il n'est pas donné plus de précision, on entend un radical alkyle linéaire ou ramifié comptant de 1 à 6 atomes de carbone et présentant au moins une double insaturation (triple liaison). Par allènyle, on entend le radical -CH=C=CH₂. Par aryle carbocyclique ou hétérocyclique, on entend un système carbocyclique (en particulier, le radical phényle qui peut être noté de façon abrégée Ph) ou hétérocyclique comprenant au moins un cycle aromatique, un système étant dit hétérocyclique lorsque l'un au moins des cycles qui le composent comporte un hétéroatome (O, N ou S). Par hétérocycle, on entend un système mono- ou polycyclique ledit système comprenant au moins un hétéroatome choisi parmi O, N et S et étant saturé, partiellement ou totalement insaturé ou aromatique. Par hétéroaryle, on entend un hétérocycle tel que défini précédemment dans lequel l'un au moins des cycles qui le composent est aromatique. Par haloalkyle, on entend un radical alkyle dont au moins l'un des atomes d'hydrogène (et éventuellement tous) est remplacé par un atome halogène.

De plus, lorsqu'il n'est pas donné plus de précision, on entend par un radical éventuellement substitué un radical comportant un ou des substituants choisis indépendamment parmi le groupe composé d'un atome halogène et des radicaux alkyle et alkoxy.

Par radicaux alkylthio, alkoxy, haloalkyle, alkoxyalkyle, trifluorométhylalkyle, cycloalkylalkyle, haloalkoxy, aminoalkyle, alkényle, alkynyle, allènylalkyle, cyanoalkyle et aralkyle, on entend respectivement les radicaux alkylthio, alkoxy, haloalkyle, alkoxyalkyle, trifluorométhylalkyle, cycloalkylalkyle, haloalkoxy, aminoalkyle, alkényle, alkynyle, allènylalkyle, cyanoalkyle et aralkyle dont le radical alkyle (les radicaux alkyle) a (ont) la (les) signification(s) indiquée(s) précédemment.

Par hétérocycle, on entend notamment les radicaux thiophène, pipéridine, pipérazine, quinoline, indoline et indole. Par alkyle linéaire ou ramifié ayant de 1 à 6 atomes de carbone, on entend en particulier les radicaux méthyle, éthyle, propyle, isopropyle, butyle, isobutyle, sec-butyle et tert-butyle, pentyle, néopentyle, isopentyle, hexyle, isohexyle. Enfin, par halogène, on entend les atomes de fluor, de chlore, de brome ou d'iode.

Plus préférentiellement, le 4-[2-(aminométhyl)-1,3-thiazol-4-yl]-2,6-di(*tert*-butyl)phénol (ou son sel pharmaceutiquement acceptable) est destiné à avoir une activité modulatrice des canaux sodiques.

En particulier, les composés des exemples 1 et 2 (parfois décrits sous forme de sels), ou leurs sels pharmaceutiquement acceptables, seront préférés lorsqu'une activité activité modulatrice des canaux sodiques sera recherchée en premier lieu.

Plus préférentiellement, le composé selon l'invention est utilisable pour préparer un médicament destiné à la fois à inhiber les MAO et la peroxydation lipidique et à moduler les canaux sodiques.

De préférence, les médicaments selon l'invention seront choisis parmi les composés décrits (parfois sous forme de sels) dans les exemples 1 à 2 ou les sels pharmaceutiquement acceptables de ces composés.

Dans certains cas, les composés selon la présente invention peuvent comporter des atomes de carbone asymétriques. Par conséquent, les composés selon la présente invention ont deux formes énantiomères possibles, c'est-à-dire les configurations "R" et "S". La présente invention inclut les deux formes énantiomères et toutes combinaisons de ces formes, y compris les mélanges racémiques "RS". Dans un souci de simplicité, lorsqu'aucune configuration spécifique n'est indiquée dans les formules de structure, il faut comprendre que les deux formes énantiomères et leurs mélanges sont représentés.

L'invention concerne également des compositions pharmaceutiques contenant, à titre de principe actif, le composé 4-[2-(aminométhyl)-1,3-thiazol-4-yl]-2,6-di(*tert*-butyl)phénol ou un sel pharmaceutiquement acceptable de ce composé, ainsi que son utilisation pour préparer un médicament destiné à inhiber les monoamine oxydases, en particulier la monoamine oxydase B, inhiber la peroxydation lipidique, avoir une activité modulatrice vis-à-vis des canaux sodiques ou à posséder deux des trois ou les trois activités précitées.

L'invention concerne de plus, à titre de médicaments, le composé 4-[2-(aminométhyl)-1,3-thiazol-4-yl]-2,6-di(*tert*-butyl)phénol ou son sel pharmaceutiquement acceptable. Elle concerne de même des compositions pharmaceutiques contenant, à titre de principe actif, le composé selon l'invention ou un sel pharmaceutiquement acceptable de ce composé.

En particulier, le composé 4-[2-(aminométhyl)-1,3-thiazol-4-yl]-2,6-di(*tert*-butyl)phénol peut être utilisé pour préparer un médicament destiné à traiter l'un des désordres ou l'une des maladies suivantes : la maladie de Parkinson, les démences séniles, la maladie d'Alzheimer, la chorée de Huntington, la sclérose latérale amyotrophique, la schizophrénie, les dépressions, les psychoses, la migraine ou les douleurs et en particulier les douleurs neuropathiques.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", *Int. J. Pharm.* (1986), **33**, 201-217.

La composition pharmaceutique peut être sous forme d'un solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection intramusculaire.

La dose d'administration envisagée pour médicament selon l'invention est comprise entre 0,1 mg à 10 g suivant le type de composé actif utilisé.

Conformément à l'invention, on peut préparer les composés de formule générale **(I)** par les procédés décrits ci-dessous.

### PRÉPARATION DES COMPOSÉS DE L'INVENTION :

### Généralités

La préparation du composé selon l'invention est effectuée de façon analogue à celles décrites dans la demande de brevet PCT WO 98/58934 (*cf. en particulier en pages 39 à 45 de ce document les synthèses des intermédiaires de formules générales (XXV) et (XXVIII)*) ou selon les procédures décrites ci-après.

### Préparation des composés de formule générale (I)

Le composé selon l'invention peut être préparé par les 3 voies synthétiques illustrées ci-dessous (schéma 1) à partir des intermédiaires de formule générale **(V), (VII)** et (**IX**), dans lesquels A, B, R¹, R², Het et n sont tels que définis ci-dessous, L est un groupe partant comme par exemple un halogène, Alk est un radical alkyle, et enfin A représente une liaison ou un radical -(CH₂)ₓ-, -CO-(CH₂)ₓ-, -(CH₂)_{y}-O- ou -C(=NH)-.

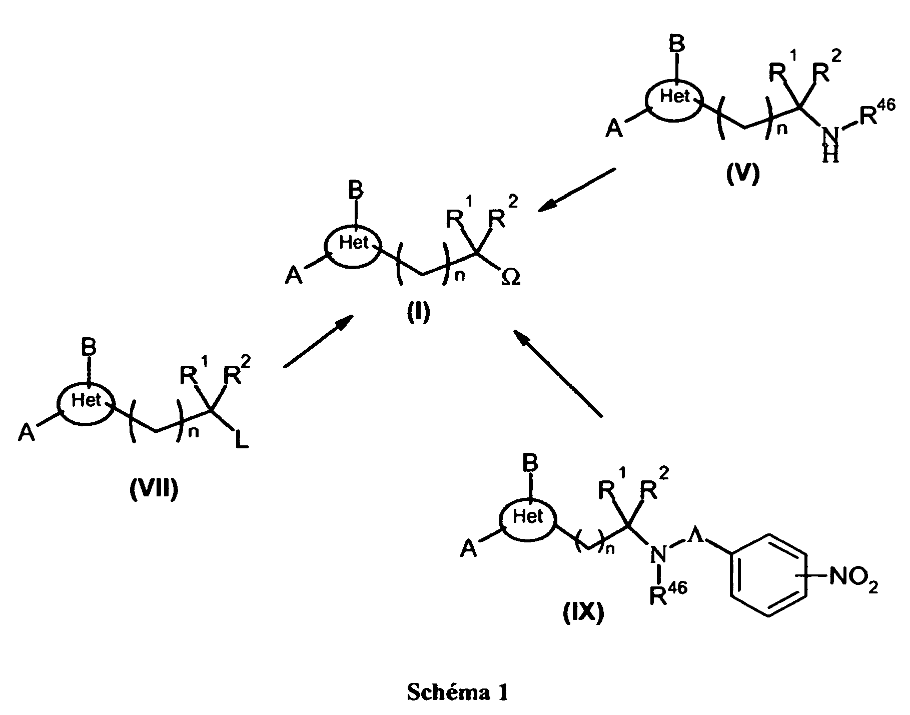

### Voie 1 : Het est thiazole et Ω est NR⁴⁶R⁴⁷

Les amines et les carboxamides de formule générale **(I),** schéma 3, dans lesquelles A, B, R¹, R², R⁴⁶, Het, g, k et n sont tels que définis ci-dessous, Δ représente un radical alkyle, cycloalkylalkyle, arylalkyle, aryle, allényle, allénylalkyle, alkényle, alkynyle, cyanoalkyle ou hydroxyalkyle et Δ' représente un radical alkyle, cycloalkylalkyle, arylalkyle ou aryle lorsque g ou k ne représente pas 0, ou Δ' représente un radical alkyle, cycloalkylalkyle, arylalkyle ou un radical aryle de préférence désactivé (c'est-à-dire un radical aryle substitué par un groupe attracteur d'électrons comme par exemple un groupe nitro ou cyano) lorsque g ou k représente 0, sont préparés par condensation des amines de formule générale (**V**) avec les acides carboxyliques (ou les chlorures d'acide correspondants) de formule générale **(XIII)** dans les conditions classiques de la synthèse peptidique, avec les aldéhydes de formule générale (**XII**) en présence d'un agent réducteur comme le triacétoxyborohydrure de sodium ou le borohydrure de sodium, dans un alcool aliphatique inférieur comme le méthanol et éventuellement en présence de tamis moléculaires, ou avec les dérivés halogénés (Hal = atome halogène) de formule générale **(XI)**. En particulier, lorsque Δ représente un radical allényle, allénylalkyle, alkényle, alkynyle, cyanoalkyle ou hydroxyalkyle, les composés de formule générale **(V)** sont convertis en les composés de formule générale **(I)** correspondants par réaction avec les dérivés halogénés de formule générale **(XI)** dans un solvant comme l'acétonitrile, le dichlorométhane ou l'acétone et en présence d'une base comme par exemple la triéthylamine ou le carbonate de potassium à une température comprise entre la température ambiante et la température de reflux du solvant.

Les dérivés de formule générale (**V**) sont notamment accessibles par une voie générale de synthèse décrite dans *Biorg. and Med. Chem. Lett.,* 1993, **3**, 915 et *Tetrahedron Lett.,* 1993. **34**, 1901, et plus particulièrement dans la demande de brevet WO 98/58934. Lorsque R⁴⁶ = H, les composés de formule générale (**V**) peuvent être préparés, par exemple, selon un protocole décrit dans la demande de brevet WO 98/58934 (en utilisant l'acide aminé adéquat à la place du N-Boc-sarcosinamide).

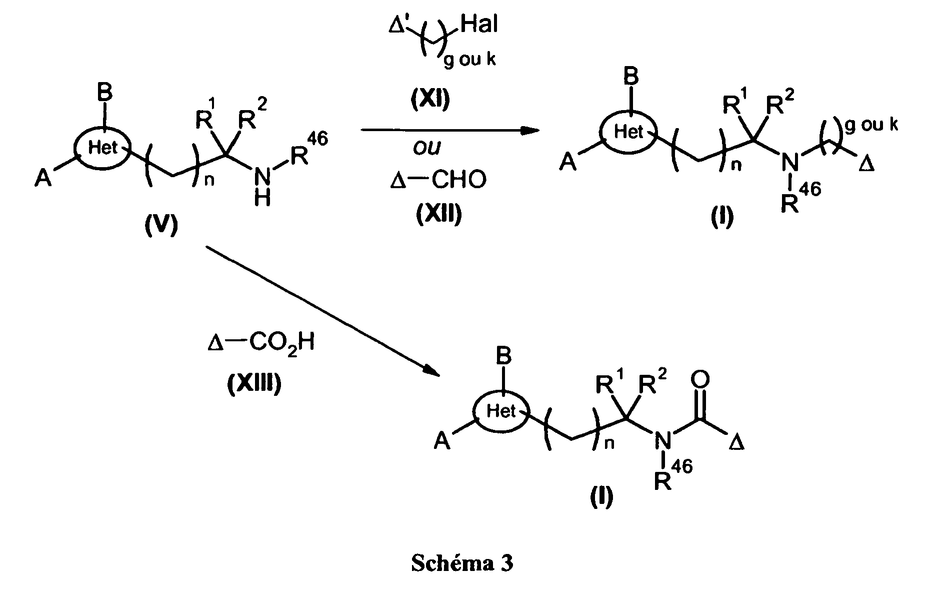

A, B, R¹, R², R⁴⁶, Het, g, k et n sont tels que définis ci-dessous :
A représente un radical 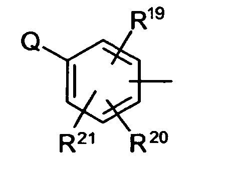 dans lequel Q représente H, -OR²², -SR²², -NR²³R²⁴, un radical phényle éventuellement substitué par un ou des substituants choisis indépendamment parmi un atome halogène, un radical OH, cyano, nitro, alkyle, alkoxy ou -NR¹⁰R¹¹ et un groupe de deux substituants représentant ensemble un radical méthylène dioxy ou éthylènedioxy, ou encore Q représente un radical -COPh, -SO₂Ph ou -CH₂Ph, ledit radical -COPh, -SO₂Ph
ou -CH₂Ph étant éventuellement substitué sur sa partie aromatique par un ou des substituants choisis indépendamment parmi un radical alkyle ou alkoxy et un atome halogène,
R¹⁰ et R¹¹ représentant, indépendamment, un atome d'hydrogène, un radical alkyle ou un groupe -COR¹², ou bien R¹⁰ et R¹¹ formant ensemble avec l'atome d'azote un hétérocycle éventuellement substitué comptant de 4 à 7 chaînons et de 1 à 3 hétéroatomes incluant l'atome d'azote déjà présent, les hétéroatomes supplémentaires étant choisis indépendamment dans le groupe constitué des atomes O, N et S, ledit hétérocycle pouvant être par exemple azétidine, pyrrolidine, pipéridine, pipérazine, morpholine ou thiomorpholine,
R¹² représentant un atome d'hydrogène, un radical alkyle ou alkoxy ou NR¹³R¹⁴,
R¹³ et R¹⁴ représentant, indépendamment, un atome d'hydrogène ou un radical alkyle, ou bien R¹³ et R¹⁴ formant ensemble avec l'atome d'azote un hétérocycle éventuellement substitué comptant de 4 à 7 chaînons et de 1 à 3 hétéroatomes incluant l'atome d'azote déjà présent, les hétéroatomes supplémentaires étant choisis indépendamment dans le groupe constitué des atomes O, N et S, ledit hétérocycle pouvant être par exemple azétidine, pyrrolidine, pipéridine, pipérazine, morpholine ou thiomorpholine,
R²² représentant un atome d'hydrogène, un radical alkyle ou un radical aryle éventuellement substitué par un ou plusieurs substituants choisis parmi les radicaux alkyle, OH, halogène, nitro et alkoxy,
R²³ et R²⁴ représentant, indépendamment, un atome d'hydrogène, un radical alkyle ou un radical -CO-R²⁵,
R²⁵ représentant un radical alkyle,
et R¹⁹, R²⁰ et R²¹ représentent, indépendamment, un hydrogène, un halogène, le groupe OH ou SR²⁶, ou un radical alkyle, cycloalkyle, alkényle, alkoxy, cyano, nitro, -SO₂NHR⁴⁹, -CONHR⁵⁵, -S(O)_{q}R⁵⁶, -NH(CO)R⁵⁷, -CF₃, -OCF₃ ou NR²⁷R²⁸,
R²⁶ représentant un atome d'hydrogène ou un radical alkyle,
R²⁷ et R²⁸ représentant, indépendamment, un atome d'hydrogène, un radical alkyle ou un groupe -COR²⁹, ou bien R²⁷ et R²⁸ formant ensemble avec l'atome d'azote un hétérocycle éventuellement substitué comptant de 4 à 7 chaînons et de 1 à 3 hétéroatomes incluant l'atome d'azote déjà présent, les hétéroatomes supplémentaires étant choisis indépendamment dans le groupe constitué des atomes O, N et S, ledit hétérocycle pouvant être par exemple azétidine, pyrrolidine, pipéridine, pipérazine, morpholine ou thiomorpholine,
R⁴⁹ et R⁵⁵ représentant, indépendamment à chaque fois qu'ils interviennent, un atome d'hydrogène ou un radical alkyle ou alkylcarbonyle,
q représentant un entier de 0 à 2,
R⁵⁶ et R⁵⁷ représentant, indépendamment à chaque fois qu'ils interviennent, un atome d'hydrogène ou un radical alkyle ou alkoxy,
R²⁹ représentant un atome d'hydrogène, un radical alkyle, alkoxy ou -NR³⁰R³¹,
R³⁰ et R³¹ représentant, indépendamment, un atome d'hydrogène ou un radical alkyle, ou bien R³⁰ et R³¹ formant ensemble avec l'atome d'azote un hétérocycle éventuellement substitué comptant de 4 à 7 chaînons et de 1 à 3 hétéroatomes incluant l'atome d'azote déjà présent, les hétéroatomes supplémentaires étant choisis indépendamment dans le groupe constitué des atomes O, N et S, ledit hétérocycle pouvant être par exemple azétidine, pyrrolidine, pipéridine, pipérazine, morpholine ou thiomorpholine,

R¹ représente un atome d'hydrogène, un radical alkyle, aminoalkyle, alkoxyalkyle, cycloalkyle, cycloalkylalkyle, trifluorométhylalkyle, alkényle, allènyle, allènylalkyle, alkynyle, cyanoalkyle, -(CH₂)_{g}-Z¹R³⁹, -(CH₂)_{g}-COR⁴⁰, -(CH₂)_{g}-NHCOR⁷⁰, aryle, aralkyle, arylcarbonyle, hétéroarylalkyle ou aralkylcarbonyle, le groupement aryle des radicaux aryle, aralkyle, arylcarbonyle, hétéroarylalkyle ou aralkylcarbonyle étant lui-même éventuellement substitué par un ou plusieurs substituants choisis parmi le groupe constitué des radicaux alkyle, halogène, alkoxy, nitro, cyano, cyanoalkyle, amino, alkylamino, dialkylamino, -(CH₂)ₖ-Z²R³⁹ ou -(CH₂)ₖ-COR⁴⁰,
Z¹ et Z² représentant une liaison, -O-, -NR⁴¹- ou -S-,
R³⁹ et R⁴¹ représentant, indépendamment à chaque fois qu'ils interviennent, un atome d'hydrogène ou un radical alkyle, alkényle, alkynyle ou cyanoalkyle,
R⁴⁰ représentant, indépendamment à chaque fois qu'il intervient, un atome d'hydrogène ou un radical alkyle, allènyle, allènylalkyle, alkényle, alkynyle, cyanoalkyle, alkoxy ou NR⁴²R⁴³,
R⁴² et R⁴³ représentant, indépendamment, indépendamment à chaque fois qu'ils interviennent, un atome d'hydrogène ou un radical alkyle, allènyle, allènylalkyle, alkényle, alkynyle ou cyanoalkyle,
et R² représente un atome d'hydrogène, un radical alkyle, aminoalkyle, alkoxyalkyle, cycloalkyle, cycloalkylalkyle, trifluorométhylalkyle ou -(CH₂)_{g}-NHCOR⁷¹, ou encore l'un des radicaux aralkyle ou hétéroarylalkyle éventuellement substitués sur le groupe aryle ou hétéroaryle par un ou des groupes choisis indépendamment parmi le groupe composé d'un atome halogène et d'un radical alkyle, alkoxy, hydroxy, cyano, nitro, amino, alkylamino ou dialkylamino,
R⁷⁰ et R⁷¹ représentant indépendamment un radical alkyle ou alkoxy ;

B représente un atome d'hydrogène, un radical alkyle, un radical -(CH₂)_{g}-Z³R⁴⁴ ou un radical aryle carbocyclique éventuellement substitué de 1 à 3 fois par des radicaux choisis parmi le groupe composé d'un atome halogène, d'un radical alkyle ou alkoxy linéaire ou ramifié comptant de 1 à 6 atomes de carbone, d'un radical hydroxy, cyano
ou nitro, d'un radical amino, alkylamino ou dialkylamino et d'un radical aryle carbocyclique,
Z³ représentant une liaison, -O-, -NR⁴⁵- ou -S-,
R⁴⁴ et R⁴⁵ représentant, indépendamment, un atome d'hydrogène ou un radical alkyle, alkényle, alkynyle, allènyle, allènylalkyle ou cyanoalkyle ;
R⁴⁶ représente un atome d'hydrogène ou un radical alkyle, cycloalkyle, cycloalkylalkyle, alkényle, alkynyle, allènyle, allènylalkyle, cyanoalkyle, -(CH₂)_{g}-Z⁴R⁵⁰, -(CH₂)ₖ-COR⁵¹, -(CH₂)ₖ-COOR⁵¹, -(CH₂)ₖ-CONHR⁵¹ ou -SO₂R⁵¹, ou encore un radical choisi parmi les radicaux aryle, aralkyle, aryloxyalkyle, arylcarbonyle, arylimino, aralkylcarbonyle, hétéroaryle et en particulier pyridinyle, pyridinylalkyle ou pyridinylcarbonyle, le groupement aryle ou hétéroaryle desdits radicaux aryle, aralkyle, aryloxyalkyle, arylcarbonyle, arylimino, aralkylcarbonyle, hétéroaryle, pyridinylalkyle ou pyridinylcarbonyle étant éventuellement substitué par un ou des substituants choisis indépendamment parmi halogène, alkyle, alkoxy, hydroxy, nitro, cyano, cyanoalkyle, amino, alkylamino, dialkylamino, -(CH₂)ₖ-Z⁵R⁵⁰, -(CH₂)ₖ-COR⁵¹ et -(CH₂)ₖ-COOR⁵¹, Z⁴ et Z⁵ représentant une liaison, -O-, -NR⁵²- ou -S-,
Het est un hétérocycle à 5 chaînons comportant 2 hétéroatomes,
g chaque fois qu'ils interviennent, étant indépendamment des entiers de 1 à 6, et k et n, chaque fois qu'ils interviennent, étant indépendamment des entiers de 0 à 6.

Dans le cas particulier où R⁴⁷ représente un radical cycloalkyle, les amines de formule générale **(I)**, schéma 3*bis*, dans laquelle A, B, R¹, R², R⁴⁶, Het et n sont tels que définis ci-dessus et i représente un entier de 0 à 4 sont préparés par condensation des amines de formule générale **(V)** avec les cycloalkylcétones de formule générale **(XIV)** en présence d'un agent réducteur comme le triacétoxyborohydrure de sodium ou le borohydrure de sodium dans un alcool aliphatique inférieur comme le méthanol et éventuellement en présence de tamis moléculaires à température ambiante.

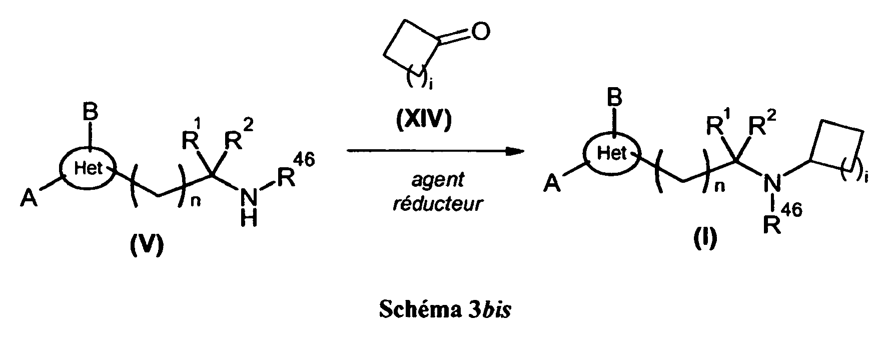

Les sulfonamides de formule générale **(I),** schéma 3*ter,* dans laquelle A, B, R¹, R², R⁴⁶, Het et n sont tels que définis ci-dessus, R⁴⁷ représente un radical -SO₂-Δ et Δ représente un radical alkyle, cycloalkyle, cycloalkylalkyle ou arylalkyle, sont préparés par condensation des amines de formule générale (**V**) avec les sulfochlorures de formule générale **(XV)** dans des conditions classiques, par exemple dans un solvant comme le diméthylformamide à température ambiante.

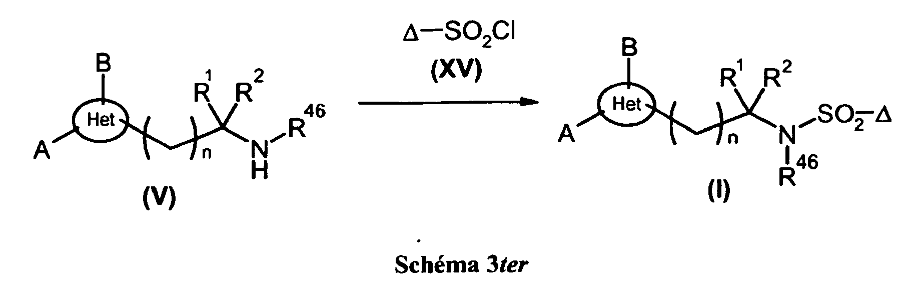

Les urées de formule générale **(I),** schéma 3*quater*, dans laquelle A, B, R¹, R², R⁴⁶, Het et n sont tels que définis ci-dessus, R⁴⁷ représente un radical -CO-NH-Δ et Δ représente un radical alkyle, cycloalkyle, cycloalkylalkyle ou arylalkyle, sont préparés par réaction des amines de formule générale **(V)** avec les isocyanates de formule générale **(XVI)** dans un solvant inerte comme le dichlorométhane ou le 1,2-dichloroéthane.

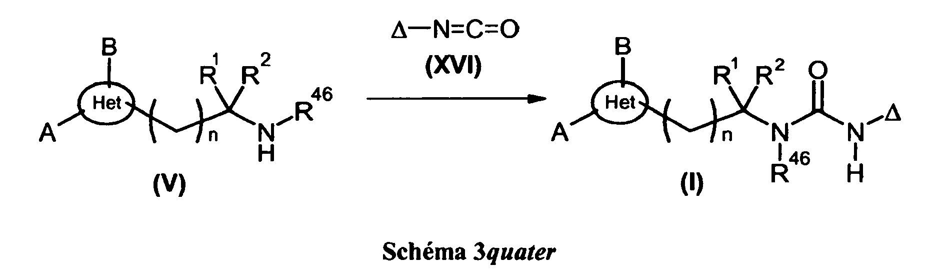

### Voie 2 : Het est thiazole et Ω est NR⁴⁶R⁴⁷

Les amines de formule générale (**I**), schéma 5, dans laquelles A, B, R¹, R², R⁴⁶, R⁴⁷, Het, et n sont tels que définis ci-dessus, sont préparées par condensation des amines primaires ou secondaires de formule générale R⁴⁶-NHR⁴⁷ avec les composés de formule générale **(VII)** (dans lesquels L représente de préférence un atome halogène Hal, mais peut aussi représenter un groupe mésylate ou tosylate) selon une voie générale de synthèse décrite dans *J*. *Med. Chem.,* 1996, **39**, 237-245 et la demande de brevet PCT WO 99/09829 ou le brevet US 4,123,529. Cette voie de synthèse peut en particulier être employée lorsque R⁴⁶ et R⁴⁷ pris ensemble forment avec l'atome d'azote qui les porte un hétérocycle non aromatique de 4 à 8 chaînons. La réaction a typiquement lieu dans un solvant anhydre (par exemple le diméthylformamide, le dichlorométhane, le tétrahydrofuranne ou l'acétone) en présence d'une base (par exemple Na₂CO₃ ou K₂CO₃ en présence de triéthylamine), et de préférence en chauffant.

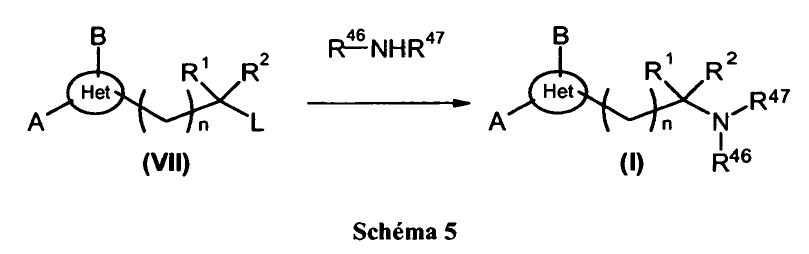

### Voie 3 : Het est thiazole et Ω est NR⁴⁶R⁴⁷

Lorsque Ω est un radical NR⁴⁶R⁴⁷ dans lequel R⁴⁷ est un radical comprenant une terminaison du type aminophénylène, alkylaminophénylène ou dialkylaminophénylène, les composés de formule générale **(I)**, dans laquelle A, B, Het, n, R¹, R² et R⁴⁶ sont tels que définis ci-dessus et A représente une liaison ou un radical -(CH₂)ₓ-, -CO-(CH₂)ₓ-, -(CH₂)_{y}-O- ou -C(=NH)-, x et y étant des entiers de 0 à 6, peuvent être obtenus, schéma 7, par réduction du composé de formule générale **(IX)**, par exemple par action d'hydrogène en présence d'un catalyseur du type palladium sur charbon dans un solvant comme par exemple le méthanol, l'éthanol, le dichlorométhane ou le tétrahydrofuranne. La réduction de la fonction nitro peut aussi être effectuée, par exemple, en chauffant le produit dans un solvant approprié tel que l'acétate d'éthyle avec un peu d'éthanol en présence de SnCl₂ (*J. Heterocyclic Chem.* (1987), **24,** 927-930 ; *Tetrahedron Letters* (1984), **25** (8), 839-842) ou en présence de SnCl₂ / Zn *(Synthesis* (1996),9,1076-1078), à l'aide de NaBH₄-BiCl₃ *(Synth. Com.* (1995) 25 (23), 3799-3803) dans un solvant tel que l'éthanol, ou alors en utilisant du Ni Raney additionné d'hydrate d'hydrazine (*Monatshefte für Chemie,* (1995), 126, 725-732), ou encore à l'aide d'indium dans un mélange d'éthanol et de chlorure d'ammonium à reflux *(Synlett (1998) 9*, 1028).

Lorsque R⁴⁷ est un radical du type aminophénylène, alkylaminophénylène ou dialkylaminophénylène (Alk et Alk' sont des radicaux alkyle identiques ou différents), le composé de formule générale **(IX)** est réduit pour conduire au dérivé aniline de formule générale **(I)** et éventuellement mono- ou di-alkylé selon des réactions classiques connues de l'homme du métier. La mono-alkylation est réalisée par amination réductrice avec un aldéhyde ou par une substitution nucléophile par réaction avec un équivalent d'halogénoalkyle Alk-Hal. Une deuxième alkylation peut ensuite être réalisée le cas échéant au moyen d'un halogénoalkyle Alk'-Hal.

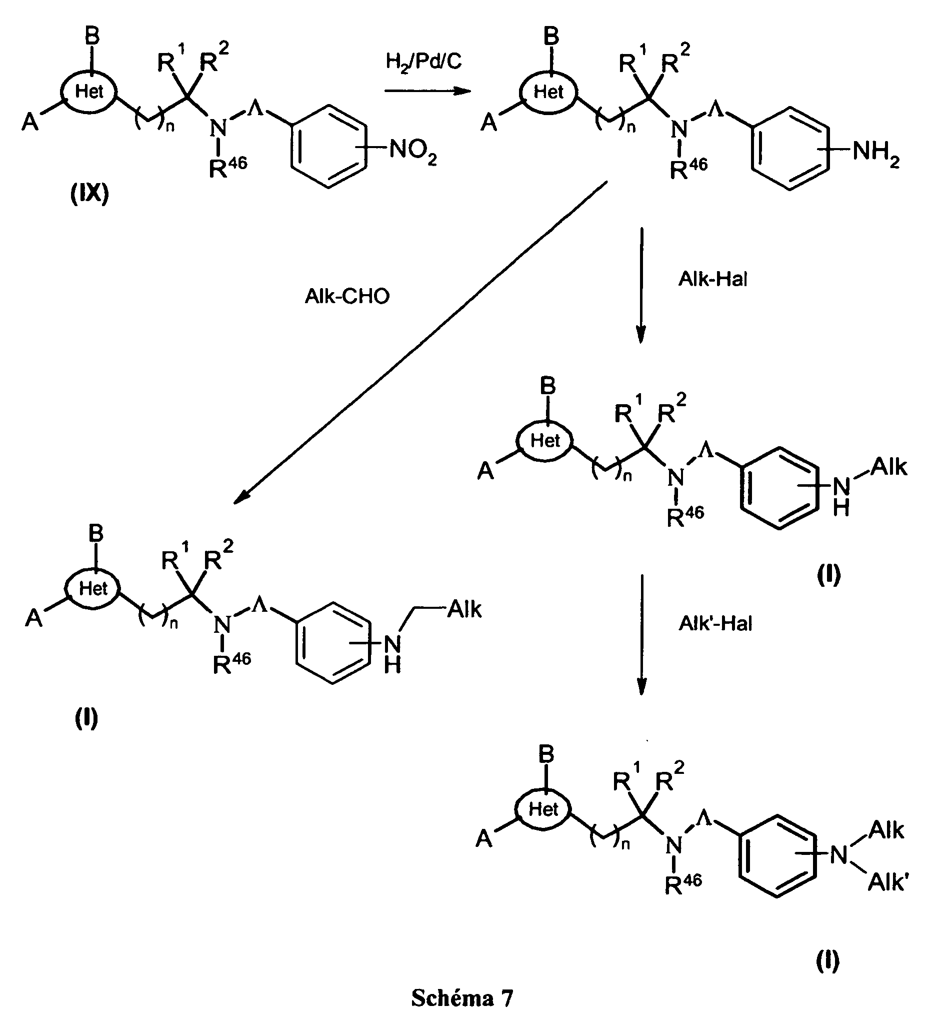

Dans le cas particulier où Alk = Alk' = -CH₃ et où A ne représente pas -CH₂-, le dérivé nitro de formule générale **(IX)** sera traité par des quantités adéquates de paraformaldéhyde sous un flux d'hydrogène dans un solvant comme l'éthanol et en présence d'un catalyseur du type palladium sur charbon (schéma *7bis).*

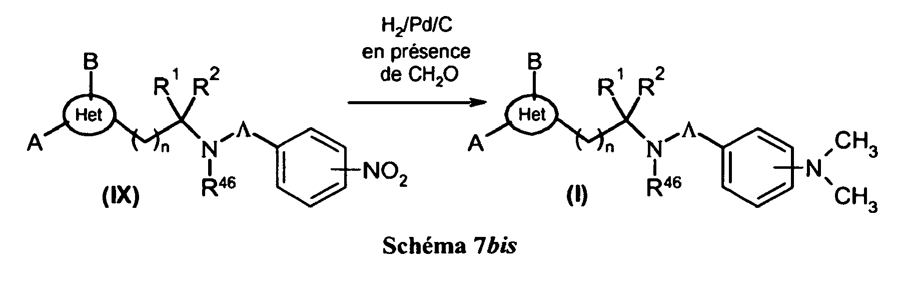

### Préparation des intermédiaires de synthèse

### Préparation des thiazoles de formule générale (V)

### Schéma général

Le dérivé cétonique non commercial de formule générale **(V.i)** ou **(V.i)**_{**2**} dans laquelle A et B sont tels que définis dans la formule générale **(I)** est converti, schéma 3.1, en l'α-bromo-cétone correspondante de formule générale **(V.ii)** ou **(V.ii)**_{**2**} par réaction avec un agent de bromation tel que CuBr₂ *(J. Org. Chem.* (1964), **29,** 3459), du brome (*J. Het. Chem.* (1988), **25**, 337), du N-bromosuccinimide *(J. Amer. Chem. Soc.* (1980), **102**, 2838) en présence d'acide acétique dans un solvant comme l'acétate d'éthyle ou le dichlorométhane, HBr ou Br₂ dans de l'éther, de l'éthanol ou de l'acide acétique (*Biorg. Med. Chem. Lett*. (1996), **6**(3), 253-258 ; *J. Med. Chem.* (1988), **31**(10), 1910-1918 ; *J. Am. Chem. Soc.* (1999), **121**, 24) ou encore à l'aide d'une résine de bromation (*J. Macromol. Sci. Chem.* (1977), **A11**, (3) 507-514). Dans le cas particulier où A est un radical p-diméthylaminophényle, il est possible d'utiliser le mode opératoire figurant dans la publication *Tetrahedron Lett.,* 1998, **39** (28), 4987. L'amine de formule générale (**V**) est ensuite obtenue selon les procédures représentées dans le 3.3 (thiazoles) ci-après.

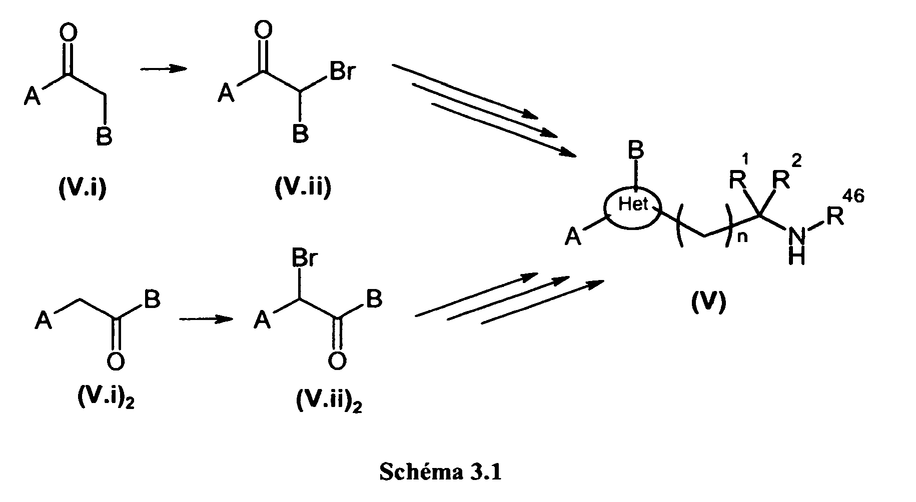

Alternativement à la synthèse présentée dans le schéma 3.1, l'homme du métier pourra, le cas échéant, utiliser une α-chloro-cétone au lieu d'une α-bromo-cétone.

### Obtention des thiazoles de formule générale (V) destinés à la préparation de composés de formule générale (I)₁ ou (I)₂.

Le thiocarboxamide de formule générale **(V.v),** dans laquelle Gp représente un groupe protecteur pour une fonction amine, par exemple un groupe protecteur de type carbamate, obtenu par exemple par réaction du carboxamide correspondant avec le réactif de Lawesson ou avec (P₂S₅)₂, est mis à réagir, schéma 3.3, avec l'α-bromo-cétone de formule générale **(V.ii)** ou **(V.ii)**_{**2**} selon un protocole expérimental décrit dans la littérature (*J. Org. Chem.,* (1995), **60,** 5638-5642). La fonction amine protégée est ensuite déprotégée dans des conditions classiques en milieu acide fort (par exemple : acide trifluoroacétique ou HCl dans un solvant organique lorsqu'il s'agit d'un groupe protecteur de type carbamate), libérant l'amine de formule générale (**V**).

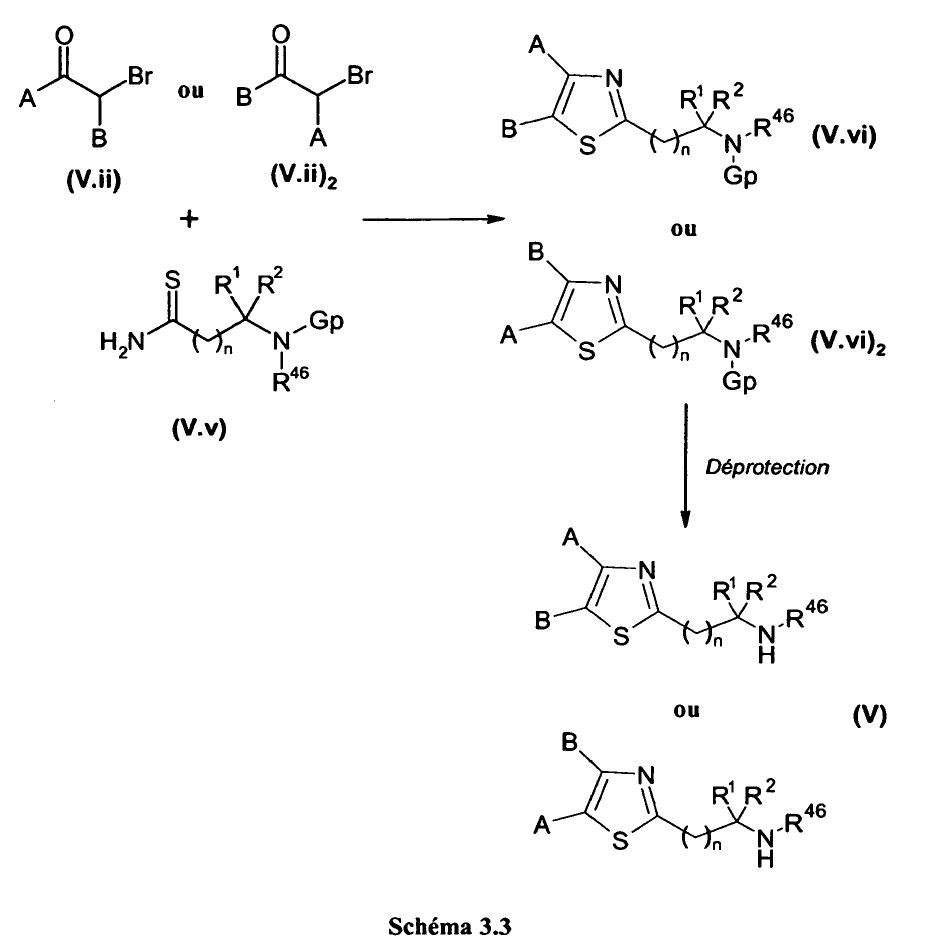

Lorsque A représente un radical phénol substitué, il peut être nécessaire de d'utiliser des intermédiaires de formule générale **(V.ii)** telle que définie précédemment dont la fonction phénol a été acétylée (ci-après désignés comme composés de formule générale **(V.ii)*ter*).** En particulier :
◆ lorsque A représente un radical 4-hydroxy-3,5-diisopropylphényle, les dérivés α-bromocétoniques homologues du composé de formule **(V.ii)** dont la fonction phénol est protégée par un radical acétyle peuvent être préparés comme résumé dans le schéma 3.13 ci-après. 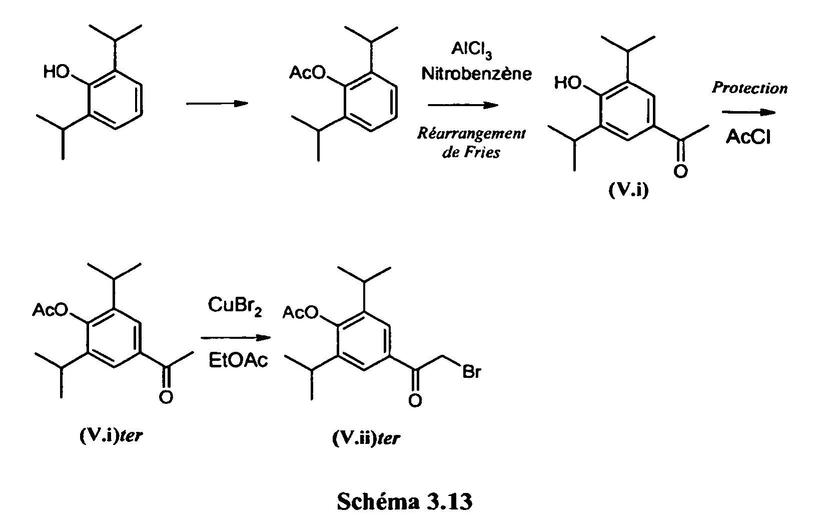 Le 2,6-düsopropylphénol est acétylé selon des méthodes connues de l'homme du métier, par exemple en le faisant réagir avec de l'acide acétique en présence d'anhydride d'acide trifluoroacétique ou avec du chlorure d'acétyle en présence d'une base comme par exemple K₂CO₃. L'homologue acétylé du 2,6-diisopropylphénol est alors soumis à un réarrangement de Fries en présence de chlorure d'aluminium dans un solvant comme le nitrobenzène pour conduire au composé de formule (**V.i**). Ensuite le composé de formule (V.i) est acétylé pour conduire au composé de formule **(V.i*)ter***. Une bromation est alors effectuée avec CuBr₂ comme précédemment décrit pour conduire au composé de formule **(V.ii)*ter*.**
   L'étape de déprotection pour libérer la fonction phénol interviendra ultérieurement dans la synthèse des composés de formule générale **(I)** (au moment jugé le plus adapté par l'homme du métier).
◆ lorsque A représente un radical de type diméthoxyphénol, les composés de formule générale **(V.ii)*ter*** peuvent être préparés de façon analogue à la synthèse décrite pour le composé de formule **(V.ii)*ter*** dérivé du 2,6-diisopropylphénol, avec éventuellement quelques modifications mineures à la portée de l'homme du métier.
   Par exemple, lorsque A représente le radical 3,5-diméthoxy-4-hydroxyphényle, le dérivé α-bromocétonique de formule **(V.ii)*ter*** correspondant peut être préparé, par exemple, comme indiqué dans le schéma 3.13 à partir du composé commercial de formule **(XXXV) :**

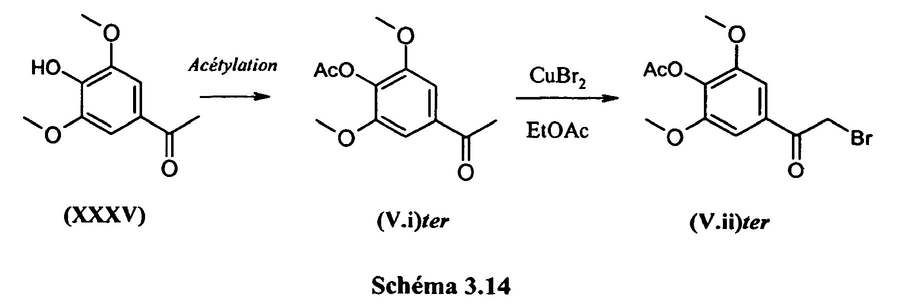

Les composés de formule générale **(V.ii)**_{**2**} dans laquelle A et B sont tels que définis précédemment peuvent être préparés selon la méthode résumée dans le schéma 3.15 ci-après.

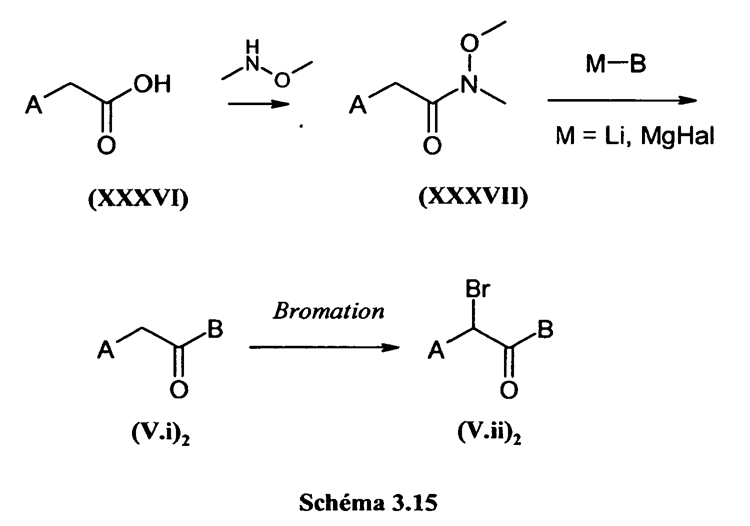

Les acides de formule générale **(XXXVI)** sont soumis à un couplage avec la N,O-diméthylhydroxylamine (*Syn. Commun.* (1995), **25,** (8), 1255 ; *Tetrahedron Lett.* (1999), **40,** (3), 411-414) dans un solvant comme le diméthylformamide ou le dichlorométhane, en présence d'une base telle que la triéthylamine avec du dicyclohexylcarbodiimide ou du chlorhydrate de 1-(3-diméthylaminopropyl)-3-éthylcarbodiimide et de l'hydroxybenzotriazole, pour conduire aux intermédiaires de formule générale **(XXXVII).** Les composés de formule générale **(V.i)**_{**2**} sont préparés à partir des composés de formule générale **(XXXVII)** par une réaction de substitution avec des dérivés lithiens ou magnésiens de formule générale B-M dans laquelle M représente Li ou MgHal (Hal = 1, Br ou Cl) dans des solvants comme l'éther ou le tétrahydrofuranne anhydre. Les α-bromo- ou α-chlorocétones de formule générale **(V.ii)**_{**2**} sont maintenant accessibles à partir des cétones de formule générale **(V.i)**_{**2**} dans des conditions précédemment décrites.

Par ailleurs, les dérivés α-halogénocétoniques de formule générale **(V.vii)** non commerciaux sont accessibles à partir de méthodes de la littérature. En particulier, ils peuvent être obtenus selon une procédure résumée dans le schéma 3.16. 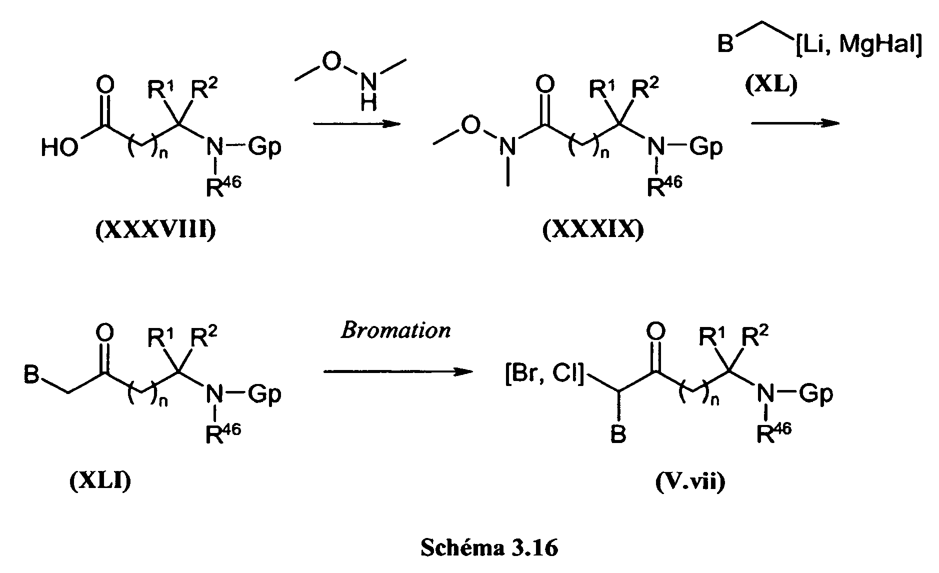 Les aminoacides protégés de formule générale **(XXXVIII)** sont obtenus par la protection des aminoacides correspondants par un groupe de type carbamate selon des méthodes connues de l'homme du métier. Les acides de formule générale **(XXXVIII)** sont ensuite soumis à un couplage avec la N,O-diméthylhydroxylamine (*Syn. Commun.* (1995), **25,** (8), 1255 ; *Tetrahedron Lett*. (1999), **40,** (3), 411-414) dans un solvant comme le diméthylformamide ou le dichlorométhane, en présence d'une base telle que la triéthylamine avec du dicyclohexylcarbodiimide ou du chlorhydrate de 1-(3-diméthylaminopropyl)-3-éthylcarbodümide et de l'hydroxybenzotriazole, pour conduire aux intermédiaires de formule générale **(XXXIX).** Les composés de formule générale **(XLI)** sont préparés à partir des composés de formule générale **(XXXIX)** par une réaction de substitution avec des dérivés lithiens ou magnésiens de formule générale **(XL)** (dans laquelle Hal = 1, Br ou Cl) dans des solvants comme l'éther ou le tétrahydrofuranne anhydre. Les bromo ou chloroacétophénones de formule générale **(V.vii)** sont maintenant accessibles à partir de l'acétophénone de formule générale **(XLI)** dans des conditions précédemment décrites.

Alternativement, l'homme du métier pourra aussi utiliser ou adapter les synthèses décrites dans *Angew. Chem. Int.* (1998), **37** (10), 411-414, *Liebigs Ann. Chem.* (1995), 1217 ou *Chem. Pharm. Bull.* (1981), **29**(11), 3249-3255.

### Préparation des dérivés acides de formule générale (V.iii).

Les dérivés acides de formule générale **(V.iii)** peuvent être obtenus, schéma 3.17, directement par réaction de l'aminoacide commercial de formule générale **(V.vi)** avec des composés de type (ar)alkylchloroformates ou di(ar)alkylcarbonates (Δ représente un radical alkyle ou benzyle) dans des conditions classiques connues de l'homme du métier.

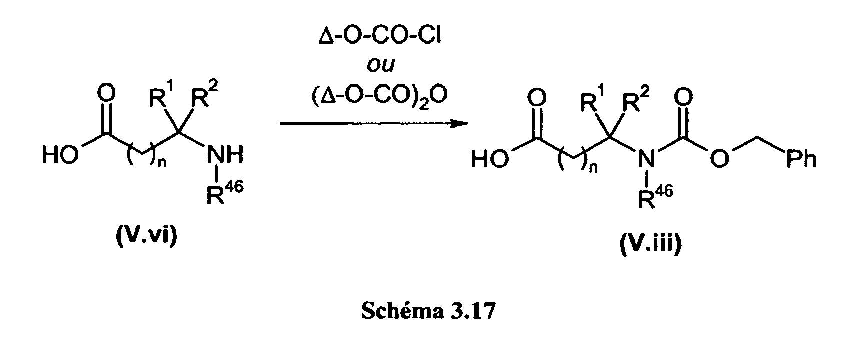

### Préparation des composés de formule générale (V.v)

Les thiocarboxamides de formule générale **(V.v)** peuvent être obtenus en trois étapes à partir des composés de formule générale **(V.vi)** comme indiqué dans le schéma 3.18 ci-dessous. La fonction amine de l'aminoacide de formule générale **(V.vi)** est d'abord protégée dans des conditions classiques avec tBu-O-CO-Cl ou (tBu-O-CO)₂O (ou d'autres groupes protecteurs connus de l'homme du métier), puis l'intermédiaire obtenu est converti en son amide correspondant par des méthodes décrites dans la littérature (cf. par exemple, *J. Chem. Soc*., *Perkin Trans. 1*, (1998), **20 ,** 3479-3484 ou la demande de brevet PCT WO 99/09829). Enfin, le carboxamide est converti en thiocarboxamide de formule générale **(V.v),** par exemple par réaction avec le réactif de Lawesson dans un solvant comme le dioxane ou le tétrahydrofuranne à une température de préférence comprise entre la température ambiante et celle du reflux du mélange, ou encore à l'aide de (P₂S₅)₂ dans des conditions classiques pour l'homme du métier.

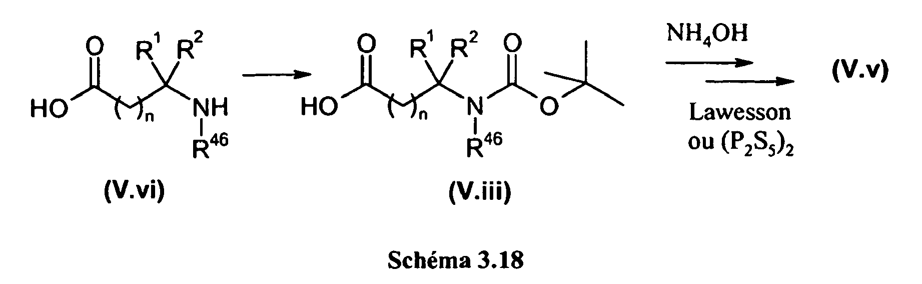

Alternativement, les thiocarboxamides de formule générale **(V.v)** peuvent également être obtenus, schéma 3.19, par addition de H₂S sur les dérivés cyano de formule générale **(V.x)** correspondants dans des conditions classiques connues de l'homme du métier.

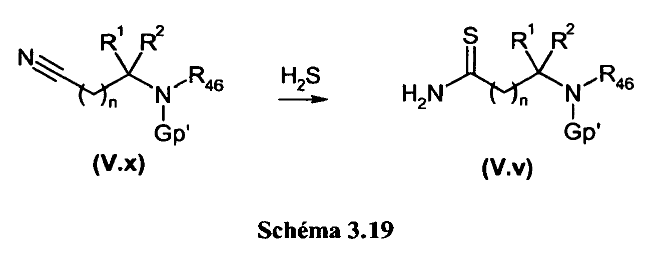

A moins qu'ils ne soient définis d'une autre manière, tous les termes techniques et scientifiques utilisés ici ont la même signification que celle couramment comprise par un spécialiste ordinaire du domaine auquel appartient cette invention.

Les exemples suivants sont présentés pour illustrer les procédures ci-dessus.

### EXEMPLES

### Exemple 1 : {4-[3,5-di(tert-butyl)-4-hydroxyphényl]-1,3-thiazol-2-yl}méthylcarbamate de benzyle :

Le composé est fabriqué selon un protocole expérimental décrit dans la demande de brevet WO 98/58934 (voir préparation des intermédiaires 26.1 et 26.2), en utilisant Z-Gly-NH₂ à la place du N-Boc sarcosinamide. Le composé attendu est obtenu sous forme d'une huile jaune pâle avec un rendement de 99%. MH+ = 453,20

### Exemple 2 : 4-[2-(aminométhyl)-1,3-thiazol-4-yl]-2,6-di(tert-butyl)phénol :

A une solution de 0,106 g (1,1 mmol) du composé de l'exemple 1 dans 10 ml de méthanol on ajoute goutte à goutte 0,1 ml d'une solution d'hydroxyde de potassium à 40%. Après une nuit d'agitation à reflux, le mélange réactionnel est concentré sous vide et le résidu est dilué avec du dichlorométhane et lavé avec une solution HCl 1*N* puis 50 ml d'une solution saturée de NaCl. La phase organique est séparée et séchée sur sulfate de magnésium, filtrée et concentrée sous vide. Le produit attendu est obtenu après chromatographie sur une colonne de silice (éluant : 5% d'éthanol dans du dichlorométhane) sous forme d'une mousse marron avec un rendement de 76%.
MH+ = 319,29.

### Etude pharmacologique des produits de l'invention

### Etude des effets sur la liaison d'un ligand spécifique de la MAO-B, le [³H]Ro 19-6327

L'activité inhibitrice des produits de l'invention est déterminée par la mesure de leurs effets sur la liaison d'un ligand spécifique de la MAO-B, le [³H]Ro 19-6327.

### a) Préparation mitochondriale de cortex de rats

La préparation mitochondriale de cortex de rats est réalisée selon la méthode décrite dans Cesura A M, Galva M D, Imhof R et Da Prada M, *J. Neurochem.* **48** (1987), 170-176. Les rats sont décapités et leurs cortex prélevés, homogénéisés dans 9 volumes d'un tampon sucrose 0,32 M tamponné à pH 7,4 avec 5 mM d'HEPES, puis centrifugés à 800 g pendant 20 minutes. Les surnageants sont récupérés et les culots lavés 2 fois avec le tampon sucrose 0,32 M comme précédemment. Les surnageants récoltés sont centrifugés à 10000 g pendant 20 minutes. Les culots obtenus sont mis en suspension dans un tampon Tris (50 mM Tris, 130 mM NaCl, 5 mM KCl, 0,5 mM EGTA, 1 mM MgCl₂, pH 7,4) et centrifugés à 10000 g pendant 20 minutes. Cette étape est répétée 2 fois, et le culot final, correspondant à la fraction mitochondriale, est conservé à -80 °C dans le tampon Tris. Le contenu protéique de la préparation est déterminé par la méthode de Lowry.

### b) Liaison du [³H]Ro 19-6327

Dans un tube Eppendorf, 100 µl de la préparation mitochondriale (2 mg protéine/ml) sont incubés pendant 1 heure à 37 °C en présence de 100 µl de [³H] Ro 19-6327 (33 nM, concentration finale) et 100 µl de tampon Tris contenant ou non les inhibiteurs. La réaction est arrêtée par l'addition de 1 ml de tampon Tris froid dans chaque tube, puis les échantillons sont centrifugés 2 minutes à 12000 g. Les surnageants sont aspirés et les culots lavés avec 1 ml de tampon Tris. Les culots sont ensuite solubilisés dans 200 µl de sodium dodécyl sulfate (20% poids/volume) pendant 2 heures à 70 °C. La radioactivité est déterminée par comptage des échantillons en scintillation liquide.

### Etude des effets sur la peroxydation lipidique du cortex cérébral de rat

L'activité inhibitrice des produits de l'invention est déterminée par la mesure de leurs effets sur le degré de peroxydation lipidique, déterminée par la concentration en malondialdéhyde (MDA). Le MDA produit par la peroxydation des acides gras insaturés est un bon indice de la peroxydation lipidique (H Esterbauer and KH Cheeseman, *Meth. Enzymol*. (1990) **186** : 407-421). Des rats mâles Sprague Dawley de 200 à 250 g (Charles River) ont été sacrifiés par décapitation. Le cortex cérébral est prélevé, puis homogénéisé au potter de Thomas dans du tampon Tris-HCl 20 mM, pH = 7,4. L'homogénat est centrifugé deux fois à 50000 g pendant 10 minutes à 4 °C. Le culot est conservé à -80 °C. Le jour de l'expérience, le culot est remis en suspension à la concentration de 1 g / 15 ml et centrifugé à 515 g pendant 10 minutes à 4 °C. Le surnageant est utilisé immédiatement pour la détermination de la peroxidation lipidique. L'homogénat de cortex cérébral de rat (500 µl) est incubé à 37 °C pendant 15 minutes en présence des composés à tester ou du solvant (10 µl). La réaction de peroxydation lipidique est initiée par l'ajout de 50 µl de FeCl₂ à 1 mM, d'EDTA à 1 mM et d'acide ascorbique à 4 mM. Après 30 minutes d'incubation à 37 °C la réaction est arrêtée par l'ajout de 50 µl d'une solution de di tertio butyl toluène hydroxylé (BHT, 0,2 %). Le MDA est quantifié à l'aide d'un test colorimétrique, en faisant réagir un réactif chromogène (R) le N-méthyl-2-phénylindole (650 µl) avec 200 µl de l'homogénat pendant 1 heure à 45 °C. La condensation d'une molécule de MDA avec deux molécules de réactif R produit un chromophore stable dont la longueur d'onde d'absorbance maximale est égale à 586 nm. (Caldwell et coll. *European J. Pharmacol*. (1995) **285**, 203-206). Les composés des exemples 1 à 2 décrits ci-dessus présentent une CI₅₀ inférieure à 10 µM.

### Test de liaison sur les canaux sodiques de cortex cérébraux de rat

Le test consiste à mesurer l'interaction des composés vis-à-vis de la liaison de la batrachotoxine tritiée sur les canaux sodiques dépendants du voltage selon le protocole décrit par Brown *(J. Neurosci.* (1986), **6**, 2064-2070).

### Préparation des homogénats de cortex cérébraux de rat

Les cortex cérébraux de rat Sprague-Dawley de 230-250 g (Charles River, France) sont prélevés, pesés et homogénéisés à l'aide d'un broyeur Potter muni d'un piston en téflon (10 allers/retours) dans 10 volumes de tampon d'isolement dont la composition est la suivante (sucrose 0,32 M ; K₂HPO₄ 5 mM ; pH 7,4). L'homogénat subit une première centrifugation à 1000 g pendant 10 minutes. Le surnageant est prélevé et centrifugé à 20000 g pendant 15 minutes. Le culot est repris dans le tampon d'isolement et centrifugé à 20000 g pendant 15 minutes. Le culot obtenu est remis en suspension dans du tampon d'incubation (HEPES 50 mM ; KCl 5,4 mM ; MgSO₄ 0,8 mM ; glucose 5,5 mM; chlorure de choline 130 mM pH 7,4) puis aliquoté et conservé à -80 °C jusqu'au jour du dosage. La concentration finale en protéines est comprise entre 4 et 8 mg/ml. Le dosage de protéines se fait par un kit commercialisé par BioRad (France).

### Mesure de la liaison de la batrachotoxine tritiée

La réaction de liaison se fait en incubant pendant 1 h 30 à 25 °C 100 µl d'homogénat de cortex de rat contenant 75 µg de protéines avec 100 µl de [³H] batrachotoxine-A 20-alpha benzoate (37,5 Ci/mmol, NEN) à 5 nM (concentration finale), 200 µl de tétrodotoxine à 1 µM (concentration finale) et de venin de scorpion à 40 µg/ml (concentration finale) et 100 µl de tampon d'incubation seul ou en présence des produits à tester aux différentes concentrations. La liaison non spécifique est déterminée en présence de 300 µM de veratridine et la valeur de cette liaison non spécifique est soustraite à toutes les autres valeurs. Les échantillons sont ensuite filtrés à l'aide d'un Brandel (Gaithersburg, Maryland, USA) en utilisant des plaques Unifilter GF/C préincubées avec 0,1 % de polyéthylène imine (20 µl/puits) et rincées 2 fois avec 2 ml de tampon de filtration (HEPES 5 mM ; CaCl₂ 1,8 mM ; MgSO₄ 0,8 mM ; chlorure de choline 130 mM ; BSA 0,01 % ; pH 7,4). Après avoir ajouté 20 µl de Microscint 0 ® , la radioactivité est comptée à l'aide d'un compteur à scintillation liquide (Topcount, Packard). La mesure est réalisée en duplicat. Les résultats sont exprimés en % de la liaison spécifique de la batrachotoxine tritiée par rapport au témoin.

### Résultats

Le composé de l'exemple 2 décrit ci-dessus présente une CI₅₀ inférieure ou égale à 1 µM. En outre, le composé de l'exemple 1 décrit ci-dessus présente une CI₅₀ inférieure
ou égale à 3,5 µM.

## Revendications

1. Composé **caractérisé en ce qu'**il s'agit du 4-[2-(aminométhyl)-1,3-thiazol-4-yl]-2,6-di(*tert*-butyl)phénol, ou sel d'un tel composé.

2. Médicament **caractérisé en ce qu'**il s'agit du 4-[2-(aminométhyl)-1,3-thiazol-4-yl]-2,6-di(*tert*-butyl)phénol ou d'un de ses sels pharmaceutiquement acceptables.

3. Composition pharmaceutique comprenant, à titre de principe actif, le 4-[2-(aminométhyl)-1,3-thiazol-4-yl]-2,6-di(*tert*-butyl)phénol ou un de ses sels pharmaceutiquement acceptables.

4. Utilisation du 4-[2-(aminométhyl)-1,3-thiazol-4-yl]-2,6-di(*tert*-butyl)phénol ou d'un de ses sels pharmaceutiquement acceptables pour préparer un médicament destiné à traiter une pathologie choisie parmi les pathologies suivantes : les troubles du système nerveux central ou périphérique, la schizophrénie, les dépressions, les psychoses, les troubles de la mémoire et de l'humeur, la migraine, les troubles du comportement, la boulimie et l'anorexie, les maladies auto-immunes et virales, l'addiction aux substances toxiques et les pathologies inflammatoires et prolifératives.

5. Utilisation selon la revendication 4, **caractérisée en ce que** les troubles du système nerveux central ou périphérique sont choisis parmi la maladie de Parkinson, les démences séniles, la maladie d'Alzheimer, la chorée de Huntington, la sclérose latérale amyotrophique, la schizophrénie, les dépressions, les psychoses, la migraine ou les douleurs.

6. Utilisation selon la revendication 4, **caractérisée en ce que** le médicament préparé est destiné à traiter les troubles du système nerveux central ou périphérique.

7. Utilisation selon la revendication 6, **caractérisée en ce que** les troubles du système nerveux central ou périphérique sont choisis parmi la maladie de Parkinson, les traumatismes cérébraux ou de la moelle épinière, l'infarctus cérébral, l'hémorragie sub-arachnoïde, l'épilepsie, le vieillissement, les démences séniles, la maladie d'Alzheimer, la chorée de Huntington, la sclérose latérale amyotrophique, les neuropathies périphériques et la douleur.

8. Utilisation selon la revendication 7, **caractérisée en ce que** le médicament préparé est destiné à traiter la maladie de Parkinson.

9. Utilisation selon la revendication 7, **caractérisée en ce que** le médicament préparé est destiné à traiter l'épilepsie.

10. Utilisation selon la revendication 7, **caractérisée en ce que** le médicament préparé est destiné à traiter la maladie d'Alzheimer.

11. Utilisation selon la revendication 7, **caractérisée en ce que** le médicament préparé est destiné à traiter la chorée de Huntington.

12. Utilisation selon la revendication 7, **caractérisée en ce que** le médicament préparé est destiné à traiter la sclérose latérale amyotrophique.

13. Utilisation selon la revendication 7, **caractérisée en ce que** le médicament préparé est destiné à traiter la douleur.

14. Utilisation selon la revendication 4, **caractérisée en ce que** le médicament préparé est destiné à traiter la schizophrénie.

15. Utilisation selon la revendication 4, **caractérisée en ce que** le médicament préparé est destiné à traiter les dépressions.

16. Utilisation selon la revendication 4, **caractérisée en ce que** le médicament préparé est destiné à traiter les psychoses.

17. Utilisation selon la revendication 4, **caractérisée en ce que** le médicament préparé est destiné à traiter la migraine.

18. Utilisation selon la revendication 4, **caractérisée en ce que** le médicament préparé est destiné à traiter les troubles du comportement.

19. Utilisation selon la revendication 4, **caractérisée en ce que** le médicament préparé est destiné à traiter la boulimie.

20. Utilisation selon la revendication 4, **caractérisée en ce que** le médicament préparé est destiné à traiter l'anorexie.

21. Utilisation selon la revendication 4, **caractérisée en ce que** le médicament préparé est destiné à traiter les pathologies inflammatoires et prolifératives.

22. Utilisation selon la revendication 4, **caractérisée en ce que** le médicament préparé est destiné à traiter les maladies auto-immunes et virales.

23. Utilisation selon la revendication 22, **caractérisée en ce que** le médicament préparé est destiné à traiter la sclérose en plaques.

## Claims

1. Compound **characterized in that** it is 4-[2-(aminomethyl)-1,3-thiazol-4-yl]-2,6-di(*tert*-butyl)phenol, or a salt of such a compound.

2. Medicament **characterized in that** it is 4-[2-(aminomethyl)-1,3-thiazol-4-yl]-2,6-di(*tert*-butyl)phenol or one of its pharmaceutically acceptable salts.

3. Pharmaceutical composition comprising as active ingredient 4-[2-(aminomethyl)-1,3-thiazol-4-yl]-2,6-di(*tert*-butyl)phenol or one of its pharmaceutically acceptable salts.

4. Use of 4-[2-(aminomethyl)-1,3-thiazol-4-yl]-2,6-di(*tert*-butyl)phenol or one of its pharmaceutically acceptable salts to prepare a medicament intended to treat a pathology chosen from among the following pathologies: disorders of the central or peripheral nervous system, schizophrenia, depression, psychoses, memory and mood disorders, migraine, behavioural disorders, bulimia and anorexia; auto-immune and viral diseases, addiction to toxic substances, and inflammatory and proliferative pathologies.

5. Use according to claim 4, **characterized in that** the disorders of the central or peripheral nervous system are chosen from Parkinson's disease, senile dementia, Alzheimer's disease, Huntington's chorea, amyotrophic lateral sclerosis, schizophrenia, depression, psychoses, migraine or pain.

6. Use according to claim 4, **characterized in that** the medicament prepared is intended to treat disorders of the central or peripheral nervous system.

7. Use according to claim 6, **characterized in that** the disorders of the central or peripheral nervous system are chosen from Parkinson's disease, cerebral or spinal cord traumatisms, cerebral infarction, subarachnoid hemorrhage, epilepsy, ageing, senile dementia, Alzheimer's disease, Huntington's chorea, amyotrophic lateral sclerosis, peripheral neuropathies and pain.

8. Use according to claim 7, **characterized in that** the medicament prepared is intended to treat Parkinson's disease.

9. Use according to claim 7, **characterized in that** the medicament prepared is intended to treat epilepsy.

10. Use according to claim 7, **characterized in that** the medicament prepared is intended to treat Alzheimer's disease.

11. Use according to claim 7, **characterized in that** the medicament prepared is intended to treat Huntington's chorea.

12. Use according to claim 7, **characterized in that** the medicament prepared is intended to treat amyotrophic lateral sclerosis

13. Use according to claim 7, **characterized in that** the medicament prepared is intended to treat pain.

14. Use according to claim 4, **characterized in that** the medicament prepared is intended to treat schizophrenia.

15. Use according to claim 4, **characterized in that** the medicament prepared is intended to treat depression.

16. Use according to claim 4, **characterized in that** the medicament prepared is intended to treat psychoses.

17. Use according to claim 4, **characterized in that** the medicament prepared is intended to treat migraine.

18. Use according to claim 4, **characterized in that** the medicament prepared is intended to treat behavioural disorders.

19. Use according to claim 4, **characterized in that** the medicament prepared is intended to treat bulimia.

20. Use according to claim 4, **characterized in that** the medicament prepared is intended to treat anorexia.

21. Use according to claim 4, **characterized in that** the medicament prepared is intended to treat inflammatory and proliferative pathologies.

22. Use according to claim 4, **characterized in that** the medicament prepared is intended to treat auto-immune and viral diseases.

23. Use according to claim 22, **characterized in that** the medicament prepared is intended to treat multiple sclerosis.

## Patentansprüche

1. Verbindung, **dadurch gekennzeichnet, dass** es sich um 4-[2-(Aminomethyl)-1,3-thiazol-4-yl]-2,6-di(*tert*-butyl)-phenol oder ein Salz einer solchen Verbindung handelt.

2. Medikament, **dadurch gekennzeichnet, dass** es sich um 4-[2-(Aminomethyl)-1,3-thiazol-4-yl]-2,6-di(*tert*-butyl)-phenol oder um eines seiner pharmazeutisch annehmbaren Salze handelt.

3. Pharmazeutische Zusammensetzung, die als Wirkstoff 4-[2-(Aminomethyl)-1,3-thiazol-4-yl]-2,6-di(*tert*-butyl)phenol oder eines seiner pharmazeutisch annehmbaren Salze umfasst.

4. Verwendung von 4-[2-(Aminomethyl)-1,3-thiazol-4-yl]-2,6-di(*tert*-butyl)phenol oder eines seiner pharmazeutisch annehmbaren Salze für die Herstellung eines Medikaments, das für die Behandlung einer aus den folgenden Krankheiten ausgewählten Krankheit bestimmt ist: Störungen des zentralen oder peripheren Nervensystems, Schizophrenie, Depressionen, Psychosen, Gedächtnis- und Gemütsstörungen, Migräne, Verhaltensstörungen, Bulimie und Anorexie, Autoimmun- und Viruserkrankungen, Abhängigkeit von toxischen Substanzen und entzündliche oder proliferative Krankheiten.

5. Verwendung nach Anspruch 4, **dadurch gekennzeichnet, dass** die Störungen des zentralen oder peripheren Nervensystems ausgewählt sind aus Parkinson-Krankheit, Altersdemenzen, Alzheimer-Krankheit, Chorea Huntington, amyotrophe Lateralsklerose, Schizophrenie, Depressionen, Psychosen, Migräne oder Schmerzen.

6. Verwendung nach Anspruch 4, **dadurch gekennzeichnet, dass** das hergestellte Medikament zur Behandlung von Störungen des zentralen oder peripheren Nervensystems bestimmt ist.

7. Verwendung nach Anspruch 6, **dadurch gekennzeichnet, dass** die Störungen des zentralen oder peripheren Nervensystems aus Parkinson-Krankheit, Hirn- oder Rückenmarkverletzungen, Hirninfarkt, subarachnoidale Hämorragie, Epilepsie, Altern, Altersdemenzen, Alzheimer-Krankheit, Chorea Huntington, amyotrophe Lateralsklerose, periphere Neuropathien und Schmerzen ausgewählt sind.

8. Verwendung nach Anspruch 7, **dadurch gekennzeichnet, dass** das hergestellt Medikament für die Behandlung der Parkinson-Krankheit bestimmt ist.

9. Verwendung nach Anspruch 7, **dadurch gekennzeichnet, dass** das hergestellte Medikament für die Behandlung der Epilepsie bestimmt ist.

10. Verwendung nach Anspruch 7, **dadurch gekennzeichnet, dass** das hergestellte Medikament für die Behandlung der Alzheimer-Krankheit bestimmt ist.

11. Verwendung nach Anspruch 7, **dadurch gekennzeichnet, dass** das hergestellt Medikament für die Behandlung der Chorea Huntington bestimmt ist.

12. Verwendung nach Anspruch 7, **dadurch gekennzeichnet, dass** das hergestellte Medikament für die Behandlung der amyotrophen Lateralsklerose bestimmt ist.

13. Verwendung nach Anspruch 7, **dadurch gekennzeichnet, dass** das hergestellte Medikament für die Behandlung von Schmerzen bestimmt ist.

14. Verwendung nach Anspruch 4, **dadurch gekennzeichnet, dass** das hergestellt Medikament für die Behandlung der Schizophrenie bestimmt ist.

15. Verwendung nach Anspruch 4, **dadurch gekennzeichnet, dass** das hergestellt Medikament für die Behandlung von Depressionen bestimmt ist.

16. Verwendung nach Anspruch 4, **dadurch gekennzeichnet, dass** das hergestellte Medikament für die Behandlung von Psychosen bestimmt ist.

17. Verwendung nach Anspruch 4, **dadurch gekennzeichnet, dass** das hergestellt Medikament für die Behandlung der Migräne bestimmt ist.

18. Verwendung nach Anspruch 4, **dadurch gekennzeichnet, dass** das hergestellte Medikament für die Behandlung von Verhaltensstörungen bestimmt ist.

19. Verwendung nach Anspruch 4, **dadurch gekennzeichnet, dass** das hergestellte Medikament für die Behandlung der Bulimie bestimmt ist.

20. Verwendung nach Anspruch 4, **dadurch gekennzeichnet, dass** das hergestellte Medikament für die Behandlung der Anorexie bestimmt ist.

21. Verwendung nach Anspruch 4, **dadurch gekennzeichnet, dass** das hergestellt Medikament für die Behandlung von entzündlichen und proliferativen Krankheiten bestimmt ist.

22. Verwendung nach Anspruch 4, **dadurch gekennzeichnet, dass** das hergestellte Medikament für die Behandlung von Autoimmun- und Viruserkrankungen bestimmt ist.

23. Verwendung nach Anspruch 22, **dadurch gekennzeichnet, dass** das hergestellt Medikament für die Behandlung der Multiple Sklerose bestimmt ist.
